# EUROPEAN PATENT SPECIFICATION

(11) **EP 2 415 390 B1**
(45) Date of publication and mention of the grant of the patent: **26.12.2012**
(21) Application number: 11175914.8
(22) Date of filing: 29.07.2011
(51) Int. Cl.: A61B 1/05

(54) **Electronic endoscope system**
Elektronisches Endoskopsystem
Système endoscope électronique

(30) Priority: 03.08.2010 JP 2010174179
(43) Date of publication of application: 08.02.2012
(73) Proprietor: Fujifilm Corporation, Minato-ku Tokyo (JP)
(72) Inventor: Ashida, Tsuyoshi, Ashigarakami-gun, Kanagawa (JP); Murayama, Jin, Ashigarakami-gun, Kanagawa (JP); Nakamura, Takayuki, Ashigarakami-gun, Kanagawa (JP); Hirata, Hidetoshi, Ashigarakami-gun, Kanagawa (JP); Hara, Kazuyoshi, Ashigarakami-gun, Kanagawa (JP); Yamakawa, Shinichi, Ashigarakami-gun, Kanagawa (JP); Iida, Takayuki, Ashigarakami-gun, Kanagawa (JP)
(74) Representative: Klunker . Schmitt-Nilson . Hirsch

(56) References cited:
- WO-A1-00/09001
- US-A1- 2009 147 078

## Description

### BACKGROUND OF THE INVENTION

### 1. Field of the invention

The present invention relates to an electronic endoscope system for capturing an image of an interior of an object using an image sensor.

### 2. Description Related to the Prior Art

An examination of an object using an electronic endoscope system is commonly performed in medical and industrial fields. The electronic endoscope system is composed of an electronic endoscope, a processing apparatus connected to the electronic endoscope, a light source apparatus, and the like. The electronic endoscope has an insert section to be inserted into an interior of the object.

The electronic endoscope has a distal portion at a distal end of the insert section. The distal portion includes an illumination window for applying illumination light to the interior of the object and a capture window for capturing an image of the interior of the object. An image sensor (an imaging device) captures an image of the interior of the object, illuminated with illumination light, through the capture window. The processing apparatus performs various processes to an imaging signal outputted from the image sensor to generate an observation image used for diagnosis. The observation image is displayed on a monitor connected to the processing apparatus. The light source apparatus has a white light source with adjustable light quantity, and supplies the illumination light to the electronic endoscope. The illumination light is guided to the distal portion through a light guide that is inserted through the electronic endoscope. The illumination light is applied to the interior of the object from the illumination window through an illumination optical system.

During the use of the electronic endoscope, temperature of the distal portion raises due to heat caused by transmission loss of the light guide and heat given off by the image sensor. As a result, dark current noise from the image sensor increases, which makes a white defective pixel (the so-called white spot) conspicuous. Thus, the observation image is deteriorated. Additionally, photoelectric conversion properties may vary with temperature. As a result, the imaging signal may be saturated.

To prevent the temperature rise in the distal portion, an electronic endoscope system provided with a temperature sensor for monitoring the temperature of the distal portion has been known (see Japanese Patent Laid Open Publication No. 63-071233 and No. 2007-117538). The electronic endoscope system controls the light quantity of the illumination light to keep the temperature of the distal portion not to exceed a predetermined value.

Japanese Patent Laid Open Publication No. 2007-252516 and No. 2008-035883 disclose electronic endoscopes each of which is provided with an LED at a distal portion. Because the LED gives off heat by emission of the illumination light, it is necessary to control or limit the light quantity of the illumination light in accordance with a temperature of the distal portion measured using a temperature sensor.

As described above, it is indispensable to measure the temperature of the distal portion and control the light quantity of the illumination light to minimize the temperature rise in the distal portion. However, installation of the temperature sensor and the signal transmission lines in the distal portion require additional space. Accordingly, the insert section, especially, the distal portion of the insert section increases in diameter. As a result, when the electronic endoscope is for medical use, physical stress of a patient increases.

When the light quantity of the illumination light is excessively reduced in accordance with the temperature of the distal portion, an observation image may become too dark for diagnosis. To control the light quantity appropriately, it is necessary to measure the temperature of the distal portion, particularly, the temperature of the image sensor as accurately as possible.

Document WO 00/09001 discloses a system according to the preamble of claim 1.

### SUMMARY OF THE INVENTION

An object of the present invention is to provide an electronic endoscope system capable of accurately detecting temperature of a distal portion of an insert section without using a temperature sensor.

In order to achieve the above and other objects, an electronic endoscope system includes an electronic endoscope, a memory, and a temperature converter. The electronic endoscope has an insertion section to be inserted into an interior of an object, an illumination section for illuminating the interior of the object, and an image sensor for capturing an image of the interior of the object being illuminated. The illumination section applies illumination light through a distal end of the insert section. The image sensor is disposed at the distal end. The image sensor has a plurality of pixels. Each of the pixels has a photoelectric conversion function. The memory stores temperature conversion information representing a relationship between a dark output value of the image sensor and a temperature of the image sensor. The temperature converter obtains the dark output value from the image sensor and determines the temperature using the temperature conversion information.

It is preferable that the electronic endoscope system further includes a light quantity controller for controlling a light quantity of the illumination light in accordance with the temperature. The dark output value is obtained every N frames of the image sensor and the N is an integer greater than or equal to 1. The temperature is determined every N frames. When the N is greater than or equal to 2, an individual dark output value of an Nth frame or an average of the individual dark output values of N frames is used as the dark output value.

It is preferable that the dark output value is obtained from the image sensor during a pause in the application of the illumination light. In this case, a dark pixel value is taken from a part of the pixels. The part of the pixels is located in a region outside of an image circle in the image sensor. An average of the taken dark pixel values is used as the dark output value.

It is preferable that the pixels are grouped into a first group and a second group. The first group is used for capturing the image of the object. The second group is used for obtaining the dark output value. The second group is shielded by a light-shield film. An average of dark pixel values taken from the respective pixels in the second group is used as the dark output value. When the temperature detection is performed every N frames, an average of the dark pixel values of the N frames may be used as the dark output value.

It is preferable that the memory is a table memory storing the temperature conversion information. The temperature for a dark output value not contained in the table memory is calculated using interpolation.

It is preferable that the light quantity controller sets an upper limit to the light quantity of the illumination light in accordance with the temperature, and the light quantity controller controls the light quantity of the illumination light not to exceed the upper limit. It is preferable that the upper limit includes a first upper limit with a high light quantity and a second upper limit with a low light quantity, and the light quantity controller sets the second upper limit as the upper limit when the temperature exceeds a first temperature that is a high temperature, and the temperature controller sets the first upper limit as the upper limit when the temperature is at or below a second temperature that is a low temperature.

In the present invention, the temperature is detected from the output of the image sensor. This eliminates the need for the temperature sensor. As a result, a structure of the electronic endoscope is simplified, and increase in diameter of the insert section is prevented.

### BRIEF DESCRIPTION OF THE DRAWINGS

The above and other objects and advantages of the present invention will be more apparent from the following detailed description of the preferred embodiments when read in connection with the accompanied drawings, wherein like reference numerals designate like or corresponding parts throughout the several views, and wherein:
Figure 1 is an external view of an electronic endoscope system;
Figure 2 is a block diagram showing an electric configuration of the electronic endoscope system;
Figure 3 is a plan view of a CMOS sensor;
Figure 4 is a block diagram showing an electric configuration of the CMOS sensor;
Figure 5 is a block diagram showing an electric configuration of an output circuit;
Figure 6 is a flow chart showing operation steps of the electronic endoscope system;
Figure 7 is a graph showing a relationship between temperature of the CMOS sensor and an upper limit to the light quantity of illumination light;
Figures 8A and 8B show how the upper limit to the light quantity is switched in accordance with a change in the temperature of the CMOS sensor;
Figure 9 is a graph with three different upper limits to the light quantity of the illumination light by way of example;
Figure 10 is a graph with an upper limit to the light quantity of the illumination light by way of example;
Figure 11 is an explanatory view showing an example of an image circle of the CMOS sensor;
Figure 12 is a block diagram of a light source device having an aperture stop mechanism;
Figure 13 is an explanatory view showing an example of the aperture stop mechanism; and
Figure 14 is a block diagram showing a configuration using a CCD.

### DESCRIPTION OF THE PREFERRED EMBODIMENTS

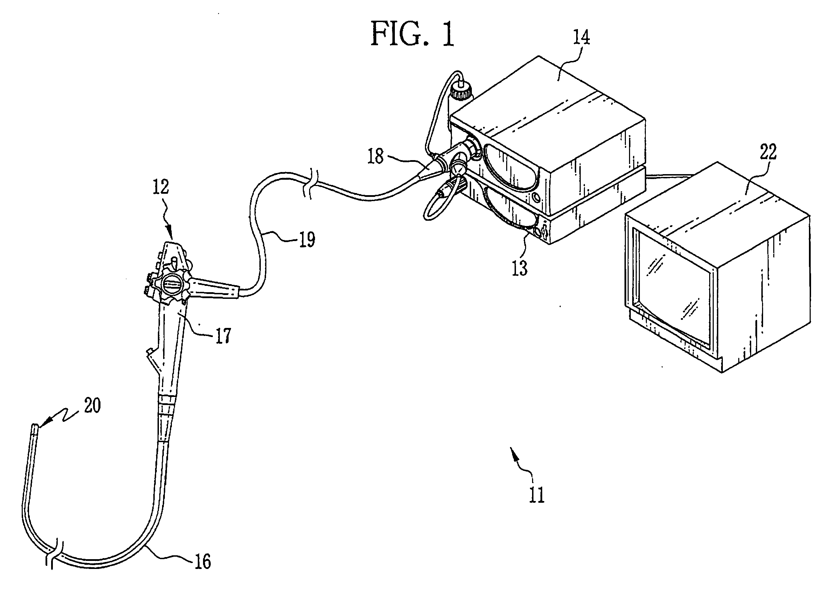

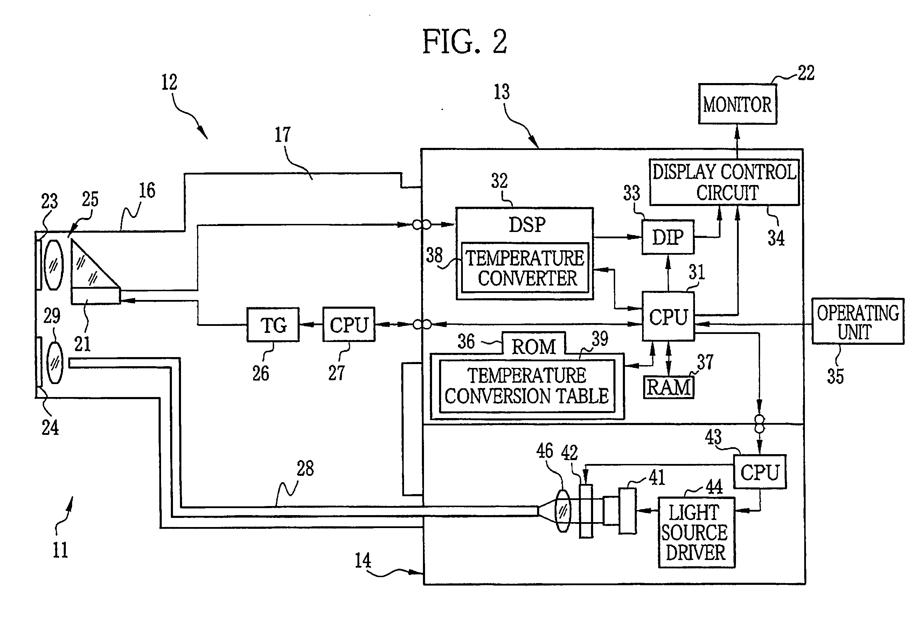

In Fig. 1, an electronic endoscope system 11 is composed of an electronic endoscope 12, a processing apparatus 13, and a light source apparatus 14. The electronic endoscope 12 is for medical use, for example, and has a flexible insert section 16 to be inserted into an interior of a patient's body, an operation section 17 connected to a base portion of the insert section 16, a connector 18 connected to the processing apparatus 13 and the light source apparatus 14, and a universal cord 19 connecting the operation section 17 and the connector 18. As shown in Fig. 2, a distal end (hereinafter referred to as the distal portion) 20 of the insert section 16 is provided with an imaging device, for example, a CMOS image sensor (hereinafter referred to as the CMOS sensor) 21.

The operation section 17 is provided with operation members such as an angle knob for directing the distal portion 20 in vertical and horizontal directions, an air/water button for ejecting air and water from a nozzle provided to the distal portion 20, a release button for recording a still observation image, and a zoom button for instructing zooming-in or zooming-out of the observation image displayed on a monitor 22. A forceps inlet is formed at an end of the operation section 17. A medical instrument such as an electrical scalpel is inserted through the forceps inlet. The forceps inlet is connected to a forceps outlet, provided in the distal portion 20, through a forceps channel across the insert section 16.

The processing apparatus 13 is electrically connected to the light source apparatus 14, and controls overall operations of the electronic endoscope system 11. The processing apparatus 13 supplies power to the electronic endoscope 12 through a transmission cable inserted through the universal cord 19 and the insert section 16 and controls the CMOS sensor 21. The processing apparatus 13 obtains an imaging signal outputted form the CMOS sensor 21 through the transmission cable. The processing apparatus 13 performs various processes to the imaging signal to generate image data. The image data is displayed as the observation image on the monitor 22 connected to the processing apparatus 13 through a cable.

As shown in Fig. 2, the distal portion 20 is provided with the forceps outlet, the air/water nozzle, a capture window 23, an illumination window 24, and the like. The CMOS sensor 21 is provided behind the capture window 23 such that an image of the interior of the patient's body is formed through an objective optical system 25. The objective optical system 25 is composed of a lens group and a prism. Illumination light is applied to the interior of the patient' s body through the illumination window 24. The light source apparatus 14 supplies the illumination light to the electronic endoscope 12. The illumination light is guided to an illumination lens 29 of the electronic endoscope 12 through a light guide 28. The light guide 28 extends throughout the universal cord 19 and in the insert section 16 of the electronic endoscope 12. The illumination lens 29 is located at an exit end of the light guide 28. The illumination light is applied to the interior of the patient's body from the illumination lens 29 through the illumination window 24.

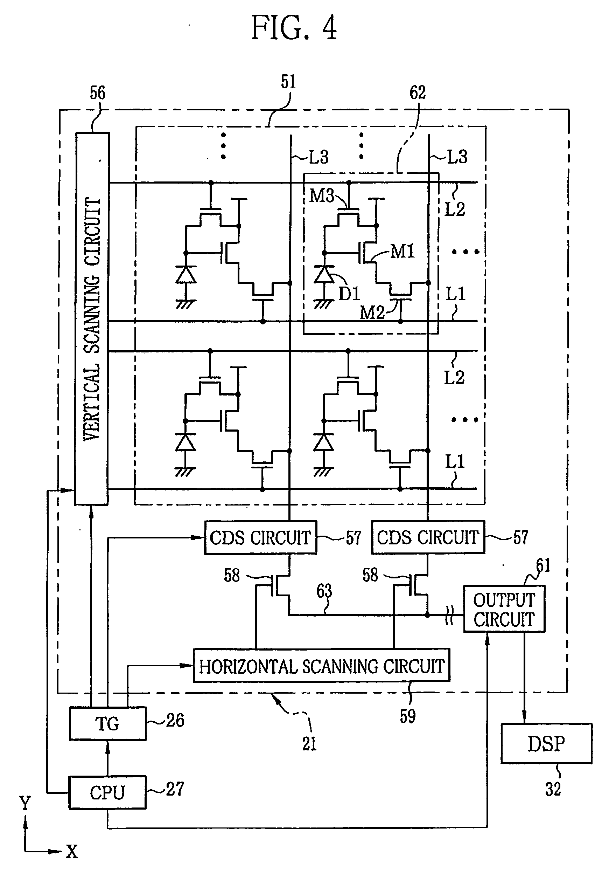

The CMOS sensor 21 is used for capturing an image of the interior of the patient' s body being illuminated. The CMOS sensor 21 has a plurality of pixels 62 arranged two-dimensionally (see Fig. 4). Each pixel 62 has a photoelectric conversion function. Each pixel 62 outputs accumulated signal charge as a pixel signal. Each pixel signal is read in a time series and forms an imaging signal.

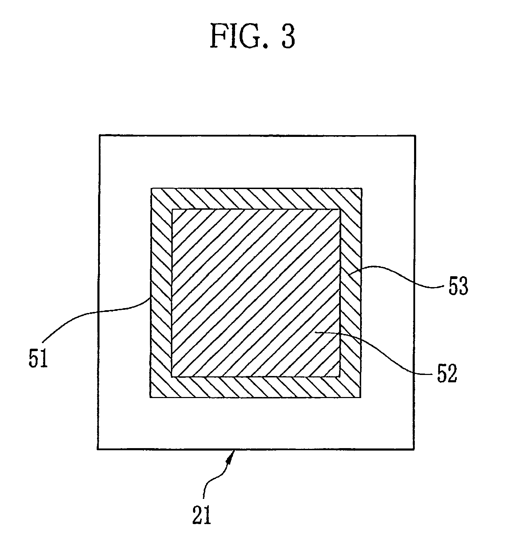

As shown in Fig. 3, the CMOS sensor 21 is provided with an imaging surface 51. The plurality of pixels 62 are arranged on the imaging surface 51. The imaging surface 51 has an effective region 52 on which light is allowed to be incident and an optical black region (hereinafter abbreviated as OB region) 53 surrounding the effective region 52. The effective region 52 and the OB region 53 are demarcated from each other. The effective region 52 is an image capturing region. Each pixel in the effective region 52 accumulates signal charge in accordance with the incident light and outputs the signal charge as an effective pixel signal at the time of reading. The OB region 53, on the other hand, is a non-image capturing region shielded with a light-shield film. The OB region 53 accumulates signal charge in accordance with a dark current and outputs the signal charge as an OB pixel signal. The dark current is also generated in a pixel in the effective region 52 and becomes noise in the effective pixel signal.

Each pixel in the effective region 52 is provided with a color filter composed of multiple color segments in a Bayer arrangement, for example. The color filter may have additive primary colors (red, green, and blue) or subtractive primary colors (cyan, magenta, and yellow, or, cyan, magenta, yellow, and green).

The CMOS sensor 21 reads the pixel signal on a line-by-line basis (a row line or a column line of the pixels 62). Accordingly, an imaging signal of one line has an effective pixel signal of the effective region 52 sandwiched between the OB pixel signals of the respective OB regions 53. An average of the OB pixel signals in each line is used for reducing the noise, caused by the dark current, from each of the effective pixel signals in the line. Furthermore, the average of the OB pixel signals of one frame, being the dark output value, is also used for detecting the temperature of the CMOS sensor 21. The temperature detection is performed on a frame-by-frame basis.

In the OB regions 53 located at the edges of the effective region 52, a scan line is formed only with the OB pixel signals. The scan line, however, is generated only in a blanking period and omitted. The OB pixel signals in the scan line may also be used to obtain the dark output value.

The operation section 17 is provided with a timing generator (hereinafter abbreviated as the TG) 26 and a CPU 27. The TG 26 provides a clock signal to the CMOS sensor 21. The CMOS sensor 21 performs imaging operation in accordance with the clock signal inputted from the TG 26, and outputs the imaging signal. The TG 26 may be provided in the CMOS sensor 21. After the electronic endoscope 12 is connected to the processing apparatus 13, the CPU 27 of the electronic endoscope 12 actuates the TG 26 based on an instruction from a CPU 31 of the processing apparatus 13.

The imaging signal outputted from the CMOS sensor 21 is inputted to the processing apparatus 13 through the universal cord 19 and the connector 18. Then, the imaging signal is temporarily stored in a working memory (not shown) of a digital signal processing circuit (hereinafter abbreviated as the DSP) 32.

The processing apparatus 13 includes the CPU 31, the DSP 32, a digital image processing circuit (hereinafter abbreviated as the DIP) 33, a display control circuit 34, an operating unit 35, and the like.

The CPU 31 of the processing apparatus 13 is connected to each section of the processing apparatus 13 through a data bus, an address bus, and control lines (all not shown) to control overall operation of the processing apparatus 13. A ROM 36 stores various programs (an OS, an application program, and the like) and data (graphic data and the like), used for controlling the operation of the processing apparatus 13. The CPU 31 reads the programs and the data from the ROM 36 and expands them in a RAM 37 that is the working memory to execute them sequentially. The CPU 31 obtains text information, such as an examination date, patient information, and operator information that vary from examination to examination, from the operating unit 35 and/or a network such as a LAN, and stores the text information in the RAM 37.

The DSP 32 performs various signal processes such as color separation, color interpolation, gain correction, white balance adjustment, and gamma correction to the effective pixel signal, out of the imaging signal from the CMOS sensor 21, to generate an image signal. The image signal generated is inputted to a working memory of the DIP 33. The DSP 32 generates data (hereinafter referred to as the ALC data) necessary for automatic light control (hereinafter abbreviated as ALC) from the pixel data, for example, and inputs the ALC data to the CPU 31. The ALC will be described later. The ALC data includes an average of brightness values of pixels, and the like.

The DSP 32 is further provided with a temperature converter 38 for detecting a temperature of the CMOS sensor 21. The temperature converter 38 obtains a dark output value from the OB pixel signal out of the imaging signal from the CMOS sensor 21. The temperature converter 38 converts the dark output value into the temperature of the CMOS sensor 21 based on the data in a temperature conversion table 39. In this embodiment, an average OB pixel value that is an average of the OB pixel signals of one frame is used as the dark output value. The temperature conversion table 39 is a data table containing or representing a relationship between the temperature of the CMOS sensor 21 and the average OB pixel value, based on actual measurements performed prior to the detection of the temperature of the CMOS sensor 21. The temperature conversion table 39 is stored in a table memory, being a part of the ROM 36. The relationship between the temperature of the CMOS sensor 21 and the average OB pixel value is not substantially affected by individual differences between the CMOS sensors 21. Instead, the average OB pixel value increases exponentially relative to the temperature rise in the CMOS sensor 21. For example, the average OB pixel value substantially doubles every 8 °C rise in temperature of the CMOS sensor 21. The temperature of the CMOS sensor 21, determined by the conversion of the average OB pixel value, is used for controlling the light quantity of the illumination light. Here, the individual differences between the CMOS sensors 21 are ignored. To be more accurate, the temperature conversion table 39 may be created individually for each CMOS sensor 21. The temperature conversion table 39 may be updated at regular maintenance or the like to reflect error or individual difference with use.

The DIP 33 performs various image processes such as electronic scaling, color enhancement, and edge enhancement to the image data generated in the DSP 32. Thereafter, the image data is inputted as the observation image to the display control circuit 34.

The display control circuit 34 has a VRAM for storing the observation image inputted from the DIP 33. The display control circuit 34 receives the graphic data and the like from the ROM 36 and the RAM 37 through the CPU 31. The graphic data and the like include a display mask, text information, and GUI. The display mask allows to display only an imaging region on which the observation image is formed, out of the effective region 52. The text information includes the examination date, the examination time, the patient information and the operator information. The display control circuit 34 superimposes the display mask, the text information, and the GUI onto the observation image stored in the VRAM, and then converts the observation image into a video signal (a component signal, a composite signal, or the like) conforming to a display format of the monitor 22, and outputs the video signal to the monitor 22. Thereby, the observation image is displayed on the monitor 22.

The operating unit 35 is a known input device such as an operation panel, a mouse, and a keyboard provided to a housing of the processing apparatus 13. The operating unit 35 also includes buttons and the like in the operation section 17 of the electronic endoscope 12. The CPU 31 of the processing apparatus 13 actuates each section of the electronic endoscope system 11 in response to an operation signal from the operating unit 35.

Additionally, the processing apparatus 13 is provided with a compression circuit, a media I/F, a network I/F, and the like. The compression circuit compresses the image data in a predetermined format (for example, JPEG format). The media I/F records the compressed image data in a removable medium in response to the operation of the release button. The network I/F controls various data transmission between the processing apparatus 13 and the network such as the LAN. The compression circuit, the media I/F, the network I/F, and the like are connected to the CPU 31 via the data bus and the like.

The light source apparatus 14 has a light source 41, a wavelength selection filter 42, and a CPU 43. The light source 41 emits light in a broad wavelength range from red to blue (for example, light in a wavelength range substantially from 400 nm to 800 nm, hereinafter simply referred to as the normal light). The light source 41 is capable of controlling the light quantity of the illumination light emitted therefrom. The light source 41 is composed of, for example, an LED or an LD, and driven by a light source driver 44. The illumination light emitted from the light source 41 is focused through a condensing lens 46 onto an incident end of the light guide 28.

Out of the illumination light from the light source 41, the wavelength selection filter 42 allows only narrowband light in a predetermined wavelength range (hereinafter referred to as the special light) to pass therethrough. The wavelength selection filter 42 is a semicircular disk rotated to be inserted or retracted from between the light source 41 and the condensing lens 46. The wavelength selection filter 42 is rotated by a motor and provided with a sensor for detecting its position. During the rotation of the wavelength selection filter 42, when the wavelength selection filter 42 is inserted between the light source 41 and the condensing lens 46, the special light is applied (the special light passes through the wavelength selection filter 42), and when the wavelength selection filter 42 is retracted from between the light source 41 and the condensing lens 46, the normal light is applied. Examples of the special light include light with a wavelength near 450 nm, 500 nm, 550 nm, 600 nm, and 780 nm.

Imaging using the special light at the wavelength near 450 nm is suitable for observation of a fine structure on a surface of a body site such as a superficial blood vessel and a pit pattern. The illumination light at the wavelength near 500 nm is suitable for macroscopic observation of recess and protrusion of a body site. The illumination light at the wavelength near 550 nm is highly absorbed by hemoglobin, so it is suitable for observation of a microvessel and flare. The illumination light at the wavelength near 600 nm is suitable for observation of hyperplasia or thickening. To observe deep blood vessels clearly, a fluorescent material such as indocyanine green (ICG) is intravenously injected, and the illumination light at the wavelength near 780 nm is applied.

Instead or in addition to the wavelength selection filter 42, LEDs or LDs emitting light in different wavelength ranges may be used as the light source 41. The LEDs and LDs may be turned on and off as necessary to switch between the normal light and the special light. Alternatively, a phosphor or fluorescent material may be used to generate normal light. When exposed to blue laser beams, the fluorescent material emits green to red excitation light. Additionally, the wavelength selection filter 42 may be used to transmit only the special light.

The CPU 43 of the light source apparatus 14 communicates with the CPU 31 of the processing apparatus 13 to control the operation of the wavelength selection filter 42. The CPU 43 functions as an automatic light control device for controlling the light source driver 44 to automatically control the light quantity of the illumination light in accordance with imaging conditions. The CPU 43 performs the automatic light control (hereinafter abbreviated as ALC) based on the ALC data generated by the DSP 32.

To perform the ALC, the CPU 43 of the light source apparatus 14 detects the temperature of the CMOS sensor 21, every frame or on a frame-by-frame basis, via the CPU 31 of the processing apparatus 13. The CPU 43 sets an upper limit to the light quantity of the illumination light outputted from the light source 41, in accordance with the temperature of the CMOS sensor 21.

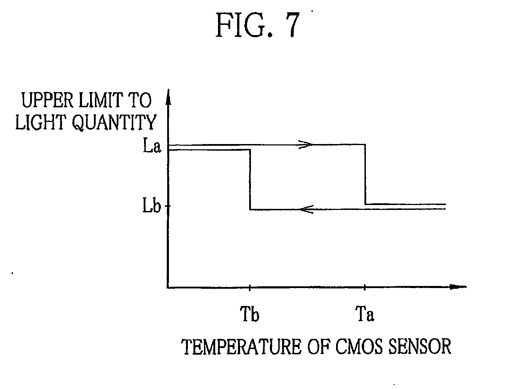

For example, as shown in Fig. 7, high and low threshold values Ta and Tb (Ta > Tb) are previously set relative to the temperature of the CMOS sensor 21, and high and low upper limits La and Lb (La > Lb) to the light quantity of the illumination light are previously set. The high and low upper limits La and Lb correspond with the high and low threshold values Ta and Tb. When the temperature of the CMOS sensor 21 exceeds the high threshold value Ta (for example, 60°C), the CPU 43 sets the low upper limit Lb as the upper limit. The ALC is performed such that the light quantity of the illumination light is within a range not exceeding the low upper limit Lb. On the other hand, when the temperature of the CMOS sensor 21 is at or below the low threshold value Tb (for example, 50°C), the CPU 43 sets the high upper limit La as the upper limit, and performs the ALC such that the light quantity of the illumination light is within a range not exceeding the high upper limit La.

The high and low threshold values Ta and Tb relative to the temperature of the CMOS sensor 21 are set to predetermined values within a range in which the normal operation of the CMOS sensor 21 is ensured (namely, within a temperature range where the white spots are inconspicuous or not noticeable). The high and low upper limits La and Lb to the light quantity of the illumination light are set on a model-by-model basis of the electronic endoscope 12. This is because the transmission loss in the light guide 28 and heat transmission to the CMOS sensor 21 vary by model of the electronic endoscope 12. In other words, the heat transmission to the CMOS sensor 21 depends on the structure inside the insert section 16, and the structure varies by the model of the electronic endoscope 12. The high and low threshold values Ta and Tb are stored in the ROM 36, for example.

The light guide 28 is, for example, a bundle of quarts optical fibers bound together using a tape. The illumination light guided to the exit end of the light guide 28 is dispersed through the illumination lens 29 and applied to the interior of the patient's body.

As shown in Fig. 4, the CMOS sensor 21 is composed of a vertical scanning circuit 56, a correlated double sampling (CDS) circuit 57, a column-selecting transistor 58, a horizontal scanning circuit 59, and an output circuit 61.

The pixels 62 are arranged in two-dimensions, for example, in a matrix on the imaging surface 51. Each of the pixels 62 has a photodiode D1, an amplifying transistor M1, a pixel-select transistor M2, and a reset transistor M3. The photodiode D1 photoelectrically converts the incident light into signal charge in accordance with the incident light quantity, and accumulates the signal charge. The signal charge accumulated in the photodiode D1 is amplified as the pixel signal by the amplifying transistor M1, and then read out at predetermined intervals by the pixel-select transistor M2. The signal charge accumulated in the photodiode D1 is transferred, at timing in accordance with the amount of the light received or charge accumulation time, to a drain through the reset transistor M3. Each of the pixel-select transistor M2 and the reset transistor M3 is a N-channel transistor that turns on when a high level "1" is applied to a gate and turns off when a low level "0" is applied to the gate.

In the imaging surface 51, a row select line L1 and a row reset line L2 are connected to the vertical scanning circuit 56 in a horizontal direction (X direction). A column signal line L3 is connected to the CDS circuit 57 in a vertical direction (Y direction). The row select line L1 is connected to a gate of the pixel-select transistor M2. The row reset line L2 is connected to a gate of the reset transistor M3. The column signal line L3 is connected to a source of the pixel-select transistor M2. The column signal line L3 is connected to the column-selecting transistor 58 of the corresponding column through the CDS circuit 57. The "rows" and "columns" are used merely to indicate relative relationships with each other.

The CDS circuit 57 holds the pixel signal from the pixel 62, connected to the row select line L1 selected by the vertical scanning circuit 56, based on a clock signal inputted from the TG 26, and removes noise from the pixel signal. The horizontal scanning circuit 59 generates a horizontal scan signal based on the clock signal inputted from the TG 26, to control turning on and off of the column-selecting transistor 58.

The column-selecting transistor 58 is provided between the CDS circuit 57 and an output bus line 63 connected to the output circuit 61. The column-selecting transistor 58 selects a pixel from which the pixel signal is transferred to the output bus line 63 in response to the horizontal scan signal.

Each of the pixel signals read out in the time series is sent as the imaging signal to the output circuit 61 through the output bus line 63. The output circuit 61 amplifies the imaging signal, and performs A/D conversion thereto, and then outputs the imaging signal as digital data. An amplification factor used for the amplification of the imaging signal is controlled by inputting a gain control signal to the output circuit 61 from the CPU 27. The output circuit 61 calculates the average OB pixel value (the average dark output value or average dark current value) from the OB pixel values of the respective pixels 62, located in the OB region 53, on a column-by-column basis of the pixels 62. The output circuit 61 subtracts the average OB pixel value from the effective pixel value of each pixel 62 located in the effective region 52. Thus, the output circuit 61 performs the dark current correction to the effective imaging signal in the effective region 52. Thereafter, the output circuit 61 performs A/D conversion to the dark-current-corrected effective imaging signal and the average OB pixel signal. The output circuit 61 outputs an imaging signal of one line, having the average OB pixel signal and the effective imaging signal aligned in this order.

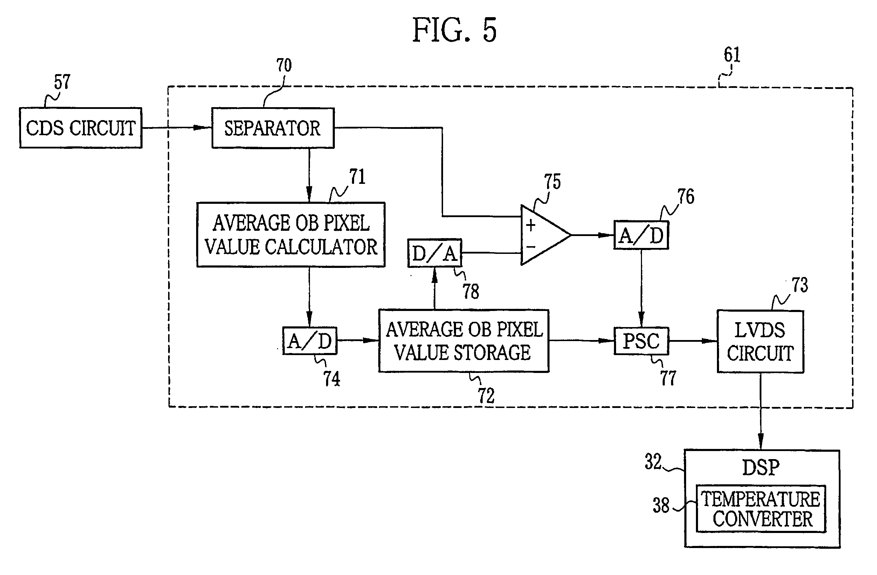

As shown in Fig. 5, the output circuit 61 has an average OB pixel value calculator 71, an average OB pixel value storage 72, and an LVDS (low voltage differential signal) circuit 73 by way of example.

The imaging signal of each line, outputted from the CDS circuit 57, is inputted to a separator 70. The separator 70 separates the imaging signal into an effective pixel signal and an OB pixel signal. The OB pixel signal is inputted to the average OB pixel value calculator 71. The average OB pixel value calculator 71 averages the OB pixel values (the dark output values or the dark current values) and calculates the average OB pixel value (the average dark output value or the average dark current values) on a line-by-line basis. An A/D converter 74 converts the average OB pixel value into digital data and then the digital average OB pixel value is temporarily stored in the average OB pixel value storage 72.

The effective pixel signal separated in the separator 70 is inputted to an amplifier 75 through a one-line delay circuit (not shown). The average OB pixel value is converted back into analog data by a D/A converter 78 and then inputted to the amplifier 75. The amplifier 75 subtracts the average OB pixel value from the effective pixel value to perform dark current correction, and then amplifies the effective pixel signal with a predetermined amplification factor. Each of the effective pixel signals outputted from the amplifier 75 in time series, that is, the effective imaging signal is converted into digital data by an A/D converter 76, and then inputted to a parallel-serial converter (PSC) 77.

When the dark-current-corrected effective imaging signal and the average OB pixel value, from the average OB pixel value storage 72, are inputted to the PSC 77, the PSC 77 produces an imaging signal in which the average OB pixel value and a plurality of the effective pixel values are aligned in this order. The imaging signal is digital data of N bit. The digital imaging signal is converted into a serial signal in which each of the N bits is serialized, and then inputted to the LVDS circuit 73.

The LVDS circuit 73 is a differential interface that uses two transmission lines to transmit a small amplitude signal. The LVDS circuit 73 transmits the imaging signal, inputted through the PSC 77, to the DSP 32. In the DSP 32, the serial imaging signal, inputted from the LVDS circuit 73, is converted into a parallel signal by a serial-parallel converter (not shown), and then received.

Next, an operation of the above-configured electronic endoscope system 11 is described. To observe the interior of the patient's body using the electronic endoscope 12, an operator connects the electronic endoscope 12, the processing apparatus 13, and the light source apparatus 14, and then turns on the processing apparatus 13 and the light source apparatus 14. The patient information and the like are inputted using the operating unit 35. The insert section 16 is inserted into the interior of the patient's body to start an examination. Upon the instruction to start the examination, the CMOS sensor 21 captures an image of the interior of the patient' s body while the illumination light (for example, the normal light) is applied through the illumination window 24 of the distal portion 20. The CMOS sensor 21 outputs an imaging signal of the captured image. The observation image is generated based on the imaging signal and displayed on the monitor 22.

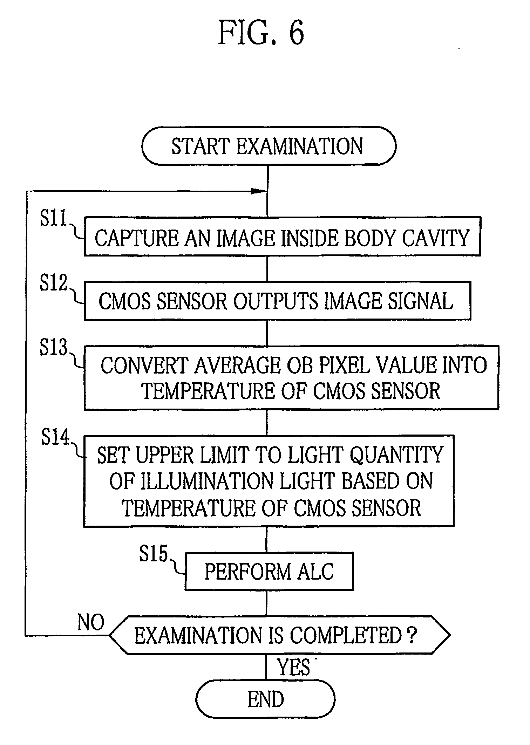

As shown in Fig. 6, when the image is captured using the electronic endoscope 12 while the interior of the patient's body is illuminated with the illumination light of a predetermined light quantity (S11), the CMOS sensor 21 outputs the imaging signal (S12). In this step, the CMOS sensor 21 calculates the average OB pixel value that is the average of the OB pixel values of the pixels 62 located in the OB region 53, out of signals outputted from the respective pixels 62 on a column-by-column basis. The CMOS sensor 21 subtracts the average OB pixel value from the effective pixel value of each of the pixels 62 located in the effective region 52 to perform the dark current correction. Then, the CMOS sensor 21 produces an imaging signal in which the average OB pixel value is added to the corrected effective pixel value of one line. The imaging signal is outputted to the DSP 32.

The DSP 32 performs various signal processes such as color separation, color interpolation, gain correction, white balance adjustment, and gamma correction to the effective pixel signal, out of the imaging signal of one line from the CMOS sensor 21, on a line-by-line basis. Thus, the image signal is generated. The image signal is inputted to the DIP 33. The DSP 32 calculates the average brightness and the like of one frame from the imaging signal generated. The average brightness and the like of one frame is used as the ALC data. The DSP 32 inputs the ALC data to the CPU 43 of the light source apparatus 14 through the CPU 31 of the processing apparatus 13. The DIP 33 performs various image processes such as the electronic scaling, the color enhancement, and the edge enhancement to the image signal inputted. Thus, the observation image is generated. The observation image is displayed on the monitor 22 through the display control circuit 34.

On the other hand, the DSP 32 calculates the average OB pixel value (the dark output value) of one frame from the average OB pixel value, out of the imaging signal of one line, using the arithmetic mean. The temperature converter 38 converts the average OB pixel value into the temperature of the CMOS sensor 21 based on the relationship between the average OB pixel value (dark output value) and the temperature stored in the temperature conversion table 39 (S13). The data of the temperature of the CMOS sensor 21 is obtained every frame, and inputted to the CPU 43 of the light source apparatus 14 that performs the ALC of the light source 41 through the CPU 31 of the processing apparatus 13.

In accordance with the temperature of the CMOS sensor 21 inputted, the CPU 43 of the light source apparatus 14 sets the upper limit, that is, one of previously-set upper limits La and Lb, to the light quantity of the illumination light used to perform the ALC (S14). The CPU 43 automatically controls the light quantity of the illumination light emitted from the light source 41 within a range not exceeding the upper limit La or Lb (S15).

The above described operation steps for the electronic endoscope system 11 are repeated until the examination is over and the image capture of the interior of the patient's body is discontinued.

As shown in Fig. 7, the relationship between the temperature of the CMOS sensor 21 and the upper limit to the light quantity of the illumination light differs between when the temperature of the CMOS sensor 21 increases and decreases. When the temperature of the CMOS sensor 21 is not high and starts to increase, for example, immediately after the start of the examination, the upper limit to the light quantity is set to the high upper limit La. The high upper limit La is maintained until the temperature of the CMOS sensor 21 reaches Ta (the high temperature threshold). When the temperature of the CMOS sensor 21 exceeds the high temperature threshold Ta, the upper limit to the light quantity is switched to the low upper limit Tb. When the temperature of the CMOS sensor 21 decreases after the upper limit to the light quantity is set to the low upper limit Lb, the low upper limit Tb is maintained until the temperature of the CMOS sensor 21 reaches Tb (the low threshold value). When the temperature of the CMOS sensor 21 is at or below the low upper limit Tb, the low upper limit Tb is switched to the high upper limit Ta.

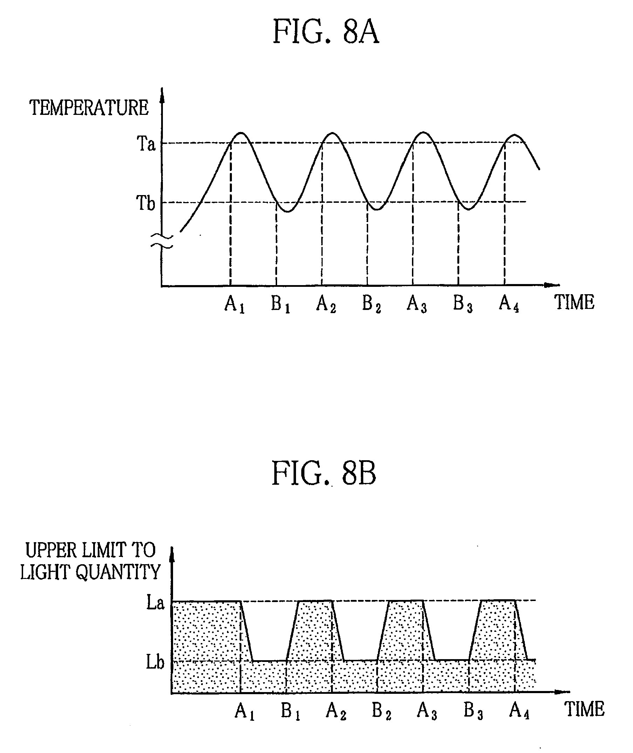

As shown in Figs. 8A and 8B, for example, from immediately after the start of the examination to a time A₁, the upper limit to the light quantity of the illumination light is set to the high upper limit La to perform the ALC. Based on the ALC data, the CPU 43 of the light source apparatus 14 automatically controls the light quantity of the illumination light within a range not exceeding the high upper limit La such that an observation image suitable for diagnosis is displayed on the monitor 22.

During the image capture inside the body cavity, when the temperature of the CMOS sensor 21 exceeds the temperature Ta at the time A₁, the CPU 43 of the light source apparatus 14 switches from the high upper limit La to the low upper limit Lb. Based on the ALC data, the CPU 43 automatically controls the light quantity of the illumination light within a range not exceeding the low upper limit Lb. Even if the light quantity of the illumination light, determined by the ALC data, necessary for capturing an observation image suitable for diagnosis exceeds the low upper limit Lb, the light quantity of the illumination light is limited not to exceed the low upper limit Lb. Thus, in the period between the time A₁ and the time B₁, for example, the light quantity of the illumination light is decreased compared to the period between the immediately after the examination and the time A₁. As a result, in the distal portion 20, the heat caused by the transmission loss of the light guide 28 is reduced.

As described above, under the automatic light control, the image capture is continued using the illumination light of the light quantity not exceeding the low upper limit Lb. When the temperature of the CMOS sensor 21 is at or below the low temperature threshold Tb at the time B₁, the CPU 43 of the light source apparatus 14 switches from the low upper limit Lb to the high upper limit La. Accordingly, in a period between the time B₁ to the time A₂, the illumination light with the light quantity higher than that between the period between the time A₁ to the time B₁ is applied in a range not exceeding the upper limit La.

Thereafter, in the same manner as the above, the CPU 43 of the light source apparatus 14 performs the ALC while switching between the upper limits of the light quantity of the illumination light in accordance with the temperature of the CMOS sensor 21. Thereby, the temperature of the CMOS sensor 21 is kept substantially between the low temperature threshold Tb and the high temperature threshold Ta even if the illumination light of the light quantity not exceeding the upper limit La or Lb is applied continuously during the image capture.

As described above, the electronic endoscope system 11 does not use a temperature sensor to detect the temperature of the distal portion 20, specifically, the temperature of the CMOS sensor 21. Instead, the electronic endoscope system 11 detects the temperature of the CMOS sensor 21 indirectly using the dark current of the CMOS sensor 21. This eliminates the need for space for the temperature sensor and signal transmission lines. Thus, it is advantageous in reducing the diameter of the insert section 16.

The temperature of the CMOS sensor 21 is detected based on the imaging signal from the CMOS sensor 21. Accordingly, the temperature of the CMOS sensor 21 is determined accurately.

The electronic endoscope system 11 switches between the high and low upper limits of the light quantity of the illumination light in accordance with the temperature of the CMOS sensor 21 during the ALC. Accordingly, the high and low threshold values Ta and Tb relative to the temperature of the CMOS sensor 21 and the high and low upper limits La and Lb to the light quantity can be set within wide ranges. When the temperature sensor is located apart from the CMOS sensor 21, the temperature measured using the temperature sensor often does not coincide with the actual temperature of the CMOS sensor 21. In this case, to surely keep the temperature of the CMOS sensor 21 not to exceed a predetermined value, the high and low threshold values Ta and Tb and the high and low upper limits La and Lb need to be set within ranges narrower than the above.

During the ALC, the electronic endoscope system 11 switches between the two upper limits to the light quantity with hysteresis relative to a change in the temperature of the CMOS sensor 21 (see Fig. 7). This prevents frequent switching between the two upper limits. Accordingly, discomfort and inconvenience, caused by hunting of the brightness of the illumination light and that of the observation image, are reduced. If there is no hysteresis and the upper limit is switched in the same condition regardless of whether the temperature of the CMOS sensor 21 increases or decreases, the switching may be repeated frequently. For example, the temperature of the CMOS sensor 21 may increase at the instant the low upper limit is switched to the high upper limit, which causes to switch from the high upper limit to the low upper limit, and vice versa.

In the above embodiment, the normal light is used as the illumination light by way of example. Alternatively, the special light may be used as the illumination light. The normal light and the special light may be used in combination or switched as necessary.

In the above embodiment, a color image sensor is used by way of example. Alternatively, a monochrome image sensor may be used. The color of the illumination light is switched to red, green, and blue sequentially using a rotating color filter to obtain the imaging signal of each color on a frame-by-frame basis (a so-called sequential method).

In the above embodiment, the temperature of the CMOS sensor 21 is determined by calculating the average OB pixel value on a frame-by-frame basis (for each frame). Because a frame rate of the CMOS sensor 21 is, for example, 60 fps or 30 fps and it is sufficiently faster than the temperature changing speed of the CMOS sensor 21, the temperature may be detected every N frames (N is an integer greater than or equal to 2), for example, every 5 frames. In this case, the average OB pixel value may also be calculated every 5 frames.

To detect the temperature every N frames, an arithmetic mean of average pixel values of respective N frames (N-frame average pixel value) may be used instead of the average OB pixel value of the Nth frame. This N-frame average pixel value further reduces the influence of the random noise. Accordingly, the temperature of the CMOS sensor 21 is detected accurately.

In the above embodiment, the average OB pixel value (the dark output value) of one frame is obtained using all the OB pixels in the OB region 53. Alternatively, a pixel value of a single OB pixel in the OB region 53 may be used as the dark output value. Alternatively, an average OB pixel value of the OB pixels in a predetermined area inside the OB region 53 may be used as the dark output value. Thereby, the calculation of the dark output value is facilitated.

In the above embodiment, the average OB pixel value is obtained on a line-by-line basis, and the dark current correction of the effective imaging signal is performed on a line-by-line basis. Alternatively, an average OB pixel value of one frame (the frame-average OB pixel value) may be obtained in advance. The effective imaging signal in the frame may be corrected using the frame-average OB pixel value. Further, because the OB pixel signals are outputted to sandwich the effective pixel signal therebetween, the average OB pixel value of the last line may be used for correcting the effective imaging signal of the next line. Furthermore, the frame-average OB pixel value of the last frame may be used for the dark current correction of the next frame.

In the above embodiment, the average OB pixel value is calculated on a line-by-line basis. Alternatively, the average OB pixel value of the entire OB region 53 may be calculated directly. Alternatively, the OB pixel value of the last line and the OB pixel value of the next line may be averaged to calculate a new average OB pixel value. For example, the average pixel value may be updated cumulatively on a line-by-line basis in one frame. Thereby, the time-varying random noise is further reduced. Accordingly, the temperature of the CMOS sensor 21 is detected accurately.

In the above embodiment, the average OB pixel value is converted into the temperature of the CMOS sensor 21 in consideration of the relationship, stored in the temperature conversion table 39, between the average OB pixel value and the temperature of the CMOS sensor 21, by way of example. The data previously stored in the temperature conversion table 39 may be discrete. When the temperature conversion table 39 does not contain a temperature of the CMOS sensor 21 that corresponds to the average OB pixel value obtained, it is preferable to calculate the corresponding temperature by interpolation using the data contained in the temperature conversion table 39. Thereby, data capacity of the temperature conversion table 39 is reduced. Furthermore, it becomes easy to perform the measurement for creating the temperature conversion table 39.

In the above embodiment, the temperature conversion table 39 is used, by way of example, as the information representing the relationship between the average OB pixel value and the temperature of the CMOS sensor 21. Instead of the temperature conversion table 39, a function expression or a function formula of the temperature of the CMOS sensor 21 relative to the average OB pixel value may be obtained. The temperature of the CMOS sensor 21 may be calculated from the average OB pixel value using the function expression.

In the above embodiment, the average OB pixel value is converted into the temperature of the CMOS sensor 21 by way of example. The relationship between the average OB pixel value and temperature of the CMOS sensor 21 is substantially constant, which allows to omit the step for converting the average OB pixel value into the temperature of the CMOS sensor 21. Namely, the upper limit to the light quantity of the illumination light may be set based only on the average OB pixel value. In the above embodiment, the temperature thresholds Ta and Tb are set relative to the temperature of the CMOS sensor 21. On the other hand, when the average OB pixel value, without the conversion into the temperature of the CMOS sensor 21, is used as a parameter for the ALC, the temperature threshold(s) may be set based on the average OB pixel value.

In the above embodiment, the two temperature thresholds Ta and Tb are set relative to the temperature of the CMOS sensor 21 and the two upper limits La and Lb are set to the light quantity of the illumination light by way of example. The number of temperature thresholds and the number of upper limits can be set as necessary.

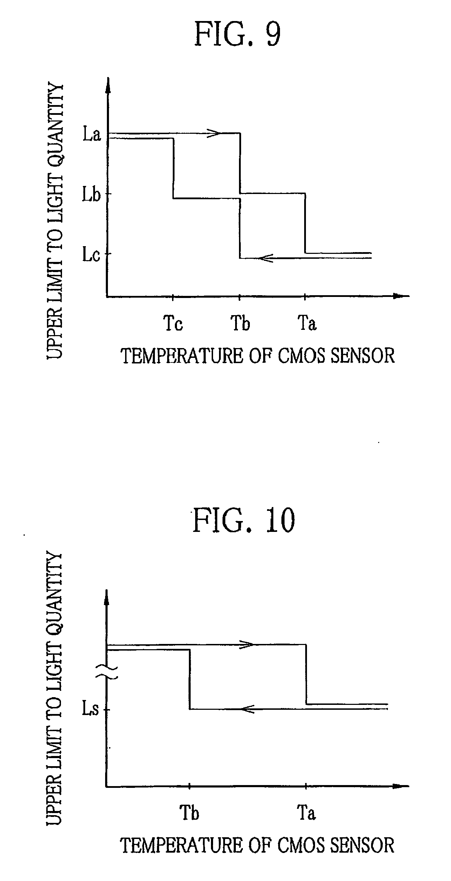

It is preferable to set three or more temperature thresholds to the temperature of the CMOS sensor 21. It is preferable to set three or more upper limits to the light quantity of the illumination light. For example, as shown in Fig. 9, three threshold values Ta, Tb, and Tc (Ta > Tb > Tc) are set relative to the temperature of the CMOS sensor 21. Three upper limits La, Lb, and Lc (La > Lb > Lc) are set relative to the light quantity of the illumination light. During the increase of the temperature T of the CMOS sensor 21, when the temperature T satisfies T < Tc, the upper limit to the light quantity is set to the maximum upper limit La. When the temperature T satisfies Tb ≤ T (< Ta), the maximum limit La is switched to the middle limit Lb. When the temperature T satisfies Ta ≤ T, the middle limit Lb is switched to the minimum upper limit Lc. To decrease the temperature T of the CMOS sensor 21, on the other hand, when the temperature T satisfies Tb < T, the upper limit to the light quantity is set to the minimum upper limit Lc. When the temperature T satisfies (Tc <) T ≤ Tb, the minimum upper limit Lc is switched to the middle limit Lb. Thereafter, when the temperature T satisfies T ≤ Tc, the middle limit Lb is switched to the maximum upper limit La. Thus, with the increased number of the temperature thresholds, the light quantity of the illumination light is adjusted more smoothly. As a result, the discomfort and inconvenience caused by the hunting of the brightness of the illumination light and that of the observation image are reduced.

In the above embodiment, the number of temperature thresholds (Ta and Tb) and the number of upper limits (La and Lb) to the light quantity are equal. Alternatively, the number of the temperature thresholds and the number of the upper limits may be different from each other. For example, as shown in Fig. 10, there are two temperature thresholds Ta and Tb and only one upper limit Ls to the light quantity of the illumination light. During the temperature increase of the CMOS sensor 21, when the temperature T of the CMOS sensor 21 satisfies T < Ta, the upper limit is removed, namely, there is no limitation up to the maximum output of the light source 41. When the temperature T satisfies Ta ≤ T, the upper limit Ls is set. On the other hand, to decrease the temperature T of the CMOS sensor 21, when the temperature T satisfies Tb < T, the upper limit Ls is set. When T ≤ Tb, the upper limit Ls is removed, namely, there is no limitation up to the maximum output of the light source 41.

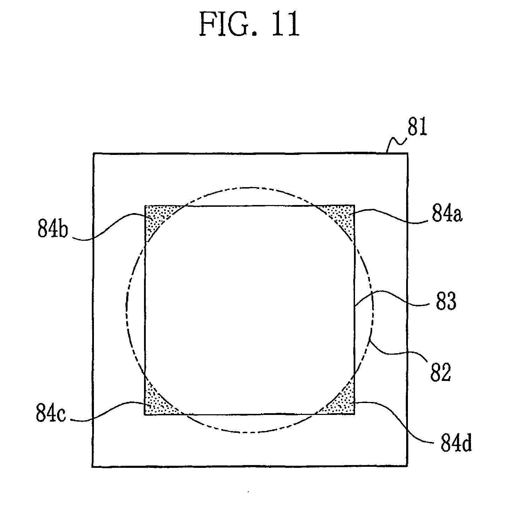

In the case where the image sensor is not provided with the OB region 53, or the image sensor does not output the data of the OB region 53, the illumination light may be applied intermittently. In a pause between the applications of the illumination light, an output signal from a pixel in the effective region 52 may be used as a dark current. For example, like a CMOS sensor 81 shown in Fig. 11, when an entire imaging surface is an effective region 83, regions 84a to 84d that are not covered by an image circle 82 of the objective optical system 25 may be used instead of the OB region 53. The output signals from the regions 84a to 84d may be taken during the illumination. It is more preferable to take the output signals during the pause between the intermittent illuminations.

In the above embodiment, the average OB pixel value is used to detect the temperature of the CMOS sensor 21. Alternatively, as a simple detection, the temperature of the CMOS sensor 21 may be detected using an effective imaging signal that is the output signal from the effective region 52.

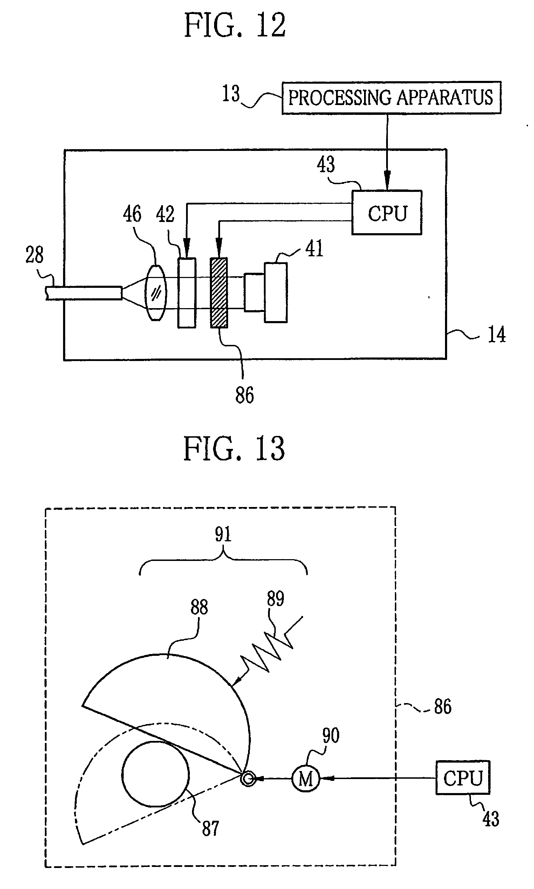

Other than the LED or LD with adjustable light quantity, for example, a xenon lamp may be used as the light source 41. The xenon lamp emits natural white light. The xenon lamp, however, needs a long time to stabilize the emission after the power is turned on. Accordingly, it is difficult to turn on and off the xenon lamp to directly control its amount of emission during the observation. In this case, as shown in Fig. 12, an aperture stop mechanism 86 is provided to the light source apparatus 14 to adjust the illumination light. The aperture stop mechanism 86 is controlled by the CPU 43 of the light source apparatus 14, and used to adjust the light quantity of the illumination light incident on the light guide 28 from the light source 41.

As shown in Fig. 13, the aperture stop mechanism 86 is provided with a diaphragm blade 88 and a spring 89. The diaphragm blade 88 covers or uncovers an aperture 87. The spring 89 biases the diaphragm blade 88 toward a position to cover the aperture 87. Against bias force of the spring 89, the torque produced by a motor (or a meter) 90 rotates the diaphragm blade 88 in a direction (clock-wise direction) to increase the opening of the aperture 87. The diaphragm blade 88 stops in a position where the torque and the bias force of the spring 89 are in balance. When the torque increases, the force against the bias force of the spring 89 also increases. Thus, the opening of the aperture 87 increases. When the torque decreases, the force against the bias force of the spring 89 also decreases. Thus, the opening of the aperture 87 decreases. The torque of the motor 90 increases with increase of a PWM (pulse width modulation) value and decreases with decrease of the PWM value.

Based on the ALC data calculated by the DSP 32, the CPU 43 of the light source apparatus 14 controls an aperture stop control mechanism 91 composed of the diaphragm blade 88 and the spring 89. In accordance with the ALC data, the CPU 43 calculates the PWM value for determining the torque of the motor 90. The motor driver (not shown) generates a drive pulse in accordance with the PWM value to drive the motor 90. The PWM value determines a duty cycle or duty ratio (pulse duration or pulse width divided by the pulse period) of the drive pulse of the motor 90. Namely, the PWM value determines the torque of the motor 90. When the ALC data is a signal requesting to increase the torque, the CPU 43 increases the PWM value accordingly. When the ALC data is a signal requesting to decrease the torque, the CPU 43 decreases the PWM value accordingly.

In the above embodiment, the known light-quantity-adjustable LED or LD is suitably used as the light source 41. For example, white light is generated by emitting light from chips of three (red, green, and blue) colors simultaneously, or by a combination of an LD (or an LED) emitting blue light and a fluorescent plate emitting yellow light when exposed to the blue light.

In the above embodiment, the light quantity of the illumination light is directly controlled in accordance with the temperature of the CMOS sensor 21. Alternatively, the amplification factor of the imaging signal may be adjusted in the output circuit 61 in accordance with the temperature of the CMOS sensor 21. Thus, the light quantity of the illumination light required by the ALC is reduced indirectly.

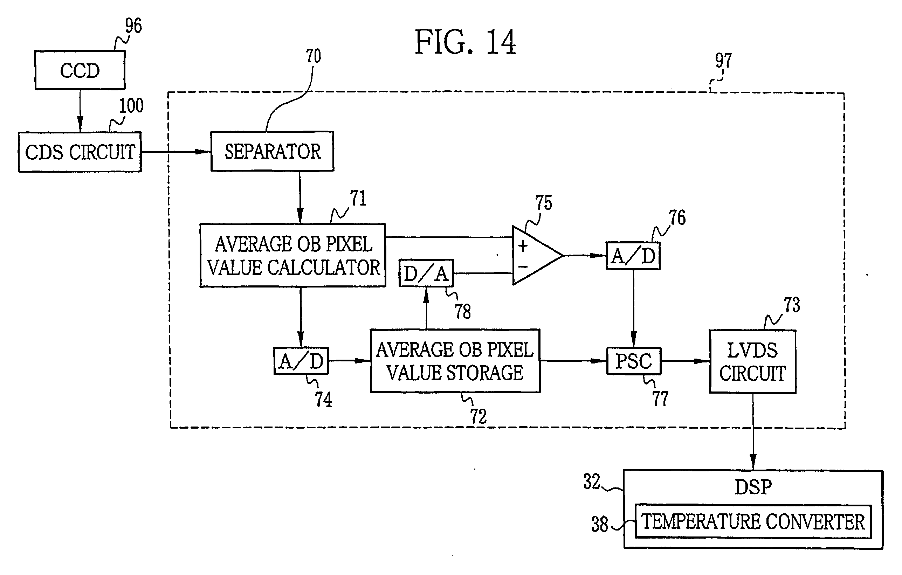

In the above embodiment, the CMOS sensor 21 is used as an example of the image sensor (the imaging device) for use in the electronic endoscope 12. Alternatively, another type of the image sensor, for example, a CCD image sensor (hereinafter referred to as the CCD) may be used. As shown in Fig. 14, when a CCD 96 is used as the image sensor, a CDS circuit 100 or the like compatible with the output circuit 61 of the CMOS sensor 21 may be provided to an analog front end (AFE) 97 for obtaining an imaging signal from the CCD 96.

In the above embodiment, the pixel 62 is composed of three transistors M1 to M3. The pixel 62 may be composed of four transistors. The pixels 62 may share the pixel-select transistor M2. The pixel 62 may have the transistors M1 and M2 located downstream from a floating diffusion section to which a signal from the photodiode D1 is transferred through a transfer transistor. The pixels 62 may share a floating diffusion section to which signals from the photodiodes D1 of the pixels 62 are transferred. The present invention is applicable to any of the above configurations.

The dark output values outputted from the respective pixels 62 in the CMOS sensor 21 vary pixel-to-pixel due to structural error caused during manufacturing process (description is omitted in the above embodiment). When the imaging signal is read out, offset correction is performed on a pixel-by-pixel basis to make the dark output values substantially equal to each other. The present invention is applicable even if the offset correction is performed to the imaging signal outputted from the CMOS sensor 21. Similarly, the offset correction is performed in the case where the CCD is used instead of the CMOS sensor 21.

Various changes and modifications are possible in the present invention and may be understood to be within the present invention.

## Claims

1. An electronic endoscope system (11) comprising:
an electronic endoscope (12) having an insertion section (16) to be inserted into an interior of an object, an illumination section (14, 28, 29) for illuminating the interior of the object, and an image sensor (21, 81) for capturing an image of the interior of the object being illuminated, the illumination section applying illumination light through a distal end (20) of the insert section, the image sensor being disposed at the distal end, the image sensor having a plurality of pixels (62), each of the pixels having a photoelectric conversion function;
**characterised by**
a memory (36) for storing temperature conversion information representing a relationship between a dark output value of the image sensor and a temperature of the image sensor; and
a temperature converter (38) adapted to obtain the dark output value from the image sensor and to determine the temperature using the temperature conversion information.

2. The electronic endoscope system of claim 1, further comprising a light quantity controller (43) for controlling a light quantity of the illumination light in accordance with the temperature.

3. The electronic endoscope system of claim 2, wherein the dark output value is obtained every N frames of the image sensor and the N is an integer greater than or equal to 1.

4. The electronic endoscope system of claim 3, wherein the temperature is determined every N frames.

5. The electronic endoscope system of claim 4, wherein when the N is greater than or equal to 2, an individual dark output value of an Nth frame or an average of the individual dark output values of N frames is used as the dark output value.

6. The electronic endoscope system of claim 4, wherein the dark output value is obtained from the image sensor during a pause in the application of the illumination light.

7. The electronic endoscope system of claim 6, wherein a dark pixel value is taken from a part of the pixels, and the part of the pixels is located in a region (84a, 84b, 84c, 84d) outside of an image circle (82) in the image sensor, and an average of the dark pixel values is used as the dark output value.

8. The electronic endoscope system of claim 4, wherein the pixels are grouped into a first group (52) and a second group (53), and the first group is used for capturing the image of the object, and the second group is used for obtaining the dark output value, and the second group is shielded by a light-shield film.

9. The electronic endoscope system of claim 8, wherein the dark output value is an average of dark pixel values taken from the respective pixels in the second group.

10. The electronic endoscope system of claim 9, wherein the dark output value is an average of the dark pixel values of the N frames.

11. The electronic endoscope system of claim 4, wherein the memory is a table memory (39) storing the temperature conversion information.

12. The electronic endoscope system of claim 11, wherein the temperature corresponding to a dark output value not contained in the table memory is calculated using interpolation.

13. The electronic endoscope system of claim 4, wherein the light quantity controller sets an upper limit to the light quantity of the illumination light in accordance with the temperature, and the light quantity controller controls the light quantity of the illumination light not to exceed the upper limit.

14. The electronic endoscope system of claim 13, wherein the upper limit includes a first upper limit with a high light quantity and a second upper limit with a low light quantity, and the light quantity controller sets the second upper limit as the upper limit when the temperature exceeds a first temperature that is a high temperature, and the temperature controller sets the first upper limit as the upper limit when the temperature is at or below a second temperature that is a low temperature.

## Patentansprüche

1. Elektronisches Endoskopsystem (11), umfassend:
ein elektronisches Endoskop (12) mit einem Einführabschnitt (16) zum Einführen in das Innere eines Objekts, einem Beleuchtungsabschnitt (14, 28, 29) zum Beleuchten des Objekt-Inneren, und einem Bildsensor (21, 81) zum Aufnehmen eines Bilds des beleuchteten Objekt-Inneren, wobei der Beleuchtungsabschnitt Beleuchtungslicht durch ein distales Ende (20) des Einführabschnitts aufbringt, der Bildsensor an dem distalen Ende angeordnet ist, wobei der Bildsensor eine Mehrzahl von Pixeln (62) aufweist, von denen jedes eine fotoelektrische Wandlerfunktion besitzt;
**gekennzeichnet durch**
einen Speicher (36) zum Speichern von Temperaturumwandlungsinformation, die eine Beziehung zwischen einem Dunkelausgabewert des Bildsensors und einer Temperatur des Bildsensors repräsentiert; und
einen Temperaturwandler (38) ausgebildet zum Ermitteln des Dunkelausgabewerts von dem Bildsensor und zum Bestimmen der Temperatur unter Verwendung der Temperaturumwandlungsinformation.

2. Elektronisches Endoskopsystem nach Anspruch 1, weiterhin umfassend eine Lichtmengensteuerung (43) zum Steuern einer Lichtmenge des Beleuchtungslichts nach Maßgabe der Temperatur.

3. Elektronisches Endoskopsystem nach Anspruch 2, bei dem der Dunkelausgabewert alle N Vollbilder des Bildsensors ermittelt wird, wobei N eine ganze Zahl größer oder gleich 1 ist.

4. Elektronisches Endoskopsystem nach Anspruch 3, bei dem die Temperatur alle N Vollbilder bestimmt wird.

5. Elektronisches Endoskopsystem nach Anspruch 4, bei dem, wenn N größer oder gleich 2 ist, ein individueller Dunkelausgabewert eines N-ten Vollbilds oder ein Durchschnittswert der individuellen Dunkelausgabewerte von N Vollbildern als Dunkelausgabewert verwendet wird.

6. Elektronisches Endoskopsystem nach Anspruch 4, bei dem der Dunkelausgabewert von dem Bildsensor erhalten wird während einer Pause des Aufbringens des Beleuchtungslichts.

7. Elektronisches Endoskopsystem nach Anspruch 6, bei dem ein Dunkelpixelwert von einem Teil der Pixel erhalten wird und der Teil der Pixel sich in einer Zone (84a, 84b, 84c, 84d) außerhalb einer Bildkreises (82) des Bildsensors befindet, und ein Durchschnittswert der Dunkelpixelwerte als der Dunkelausgabewert verwendet wird.

8. Elektronisches Endoskopsystem nach Anspruch 4, bei dem die Pixel gruppiert sind zu einer ersten Gruppe (52) und einer zweiten Gruppe (53), und die erste Gruppe zum Aufnehmen des Bilds des Objekts verwendet wird, und die zweite Gruppe dazu verwendet wird, den Dunkelausgabewert zu ermitteln, wobei die zweite Gruppe durch einen Lichtabschirmungsfilm abgeschirmt ist.

9. Elektronisches Endoskopsystem nach Anspruch 8, bei dem der Dunkelausgabewert ein Durchschnittswert von Dunkelpixelwerten ist, die von den jeweiligen Pixeln der zweiten Gruppe genommen werden.

10. Elektronisches Endoskopsystem nach Anspruch 9, bei dem der Dunkelausgabewert ein Durchschnitt der Dunkelpixelwerte von den N Vollbildern ist.

11. Elektronisches Endoskopsystem nach Anspruch 4, bei dem der Speicher ein Tabellenspeicher (39) ist, welcher die Temperaturwandlerinformation speichert.

12. Elektronisches Endoskopsystem nach Anspruch 11, bei dem die Temperatur entsprechend einem Dunkelausgabewert, der nicht in dem Tabellenspeicher enthalten ist, durch Interpolation berechnet wird.

13. Elektronisches Endoskopsystem nach Anspruch 4, bei dem die Lichtmengensteuerung die Obergrenze für die Lichtmenge des Beleuchtungslichts nach Maßgabe der Temperatur einstellt, und die Lichtmengensteuerung die Lichtmenge des Beleuchtungslichts so einstellt, dass sie die Obergrenze nicht überschreitet.

14. Elektronisches Endoskopsystem nach Anspruch 13, bei dem die Obergrenze mit einer hohen Lichtmenge und eine zweite Obergrenze mit einer geringen Lichtmenge enthält, und die Lichtmengensteuerung die zweite obere Grenze als die Obergrenze einstellt, wenn die Temperatur eine erste Temperatur übersteigt, bei der es sich um eine hohe Temperatur handelt, und die Temperatursteuerung die erste Obergrenze als die obere Grenze einstellt, wenn die Temperatur einer zweiten Temperatur, die eine geringe Temperatur ist, gleicht oder unter dieser liegt.

## Revendications

1. Système endoscopique électronique (11) comprenant :
un endoscope électronique (12) comportant une section d'insertion (16) à insérer à l'intérieur d'un objet, une section d'éclairage (14, 28, 29) pour éclairer l'intérieur de l'objet, et un capteur d'image (21, 81) pour capturer une image de l'intérieur de l'objet éclairé, la section d'éclairage appliquant une lumière d'éclairage à travers une extrémité distale (20) de la section d'insertion, le capteur d'image étant disposé à l'extrémité distale, le capteur d'image comportant une pluralité de pixels (62), chacun des pixels ayant une fonction de conversion photoélectrique ;
**caractérisé par**
une mémoire (36) pour mémoriser des informations de conversion de température représentant une relation entre une valeur de sortie d'obscurité du capteur d'image et une température du capteur d'image ; et
un convertisseur de température (38) conçu pour obtenir la valeur de sortie d'obscurité du capteur d'image et pour déterminer la température en utilisant les informations de conversion de température.

2. Système endoscopique électronique selon la revendication 1, comprenant en outre un contrôleur de quantité de lumière (43) pour commander une quantité de lumière de la lumière d'éclairage en fonction de la température.

3. Système endoscopique électronique selon la revendication 2, dans lequel la valeur de sortie d'obscurité est obtenue toutes les N trames du capteur d'image et N est un entier supérieur ou égal à 1.

4. Système endoscopique électronique selon la revendication 3, dans lequel la température est déterminée toutes les N trames.

5. Système endoscopique électronique selon la revendication 4, dans lequel, lorsque N est supérieur ou égal à 2, une valeur de sortie d'obscurité individuelle d'une n^{ième} trame ou une moyenne des valeurs de sortie d'obscurité individuelles de N trames est utilisée en tant que valeur de sortie d'obscurité.

6. Système endoscopique électronique selon la revendication 4, dans lequel la valeur de sortie d'obscurité est obtenue à partir du capteur d'image pendant une pause dans l'application de la lumière d'éclairage.

7. Système endoscopique électronique selon la revendication 6, dans lequel une valeur de pixel d'obscurité est prise à partir d'une partie des pixels, et la partie des pixels est située dans une région (84a, 84b, 84c, 84d) à l'extérieur d'un cercle d'image (82) dans le capteur d'image, et une moyenne des valeurs de pixel d'obscurité est utilisée en tant que valeur de sortie d'obscurité.

8. Système endoscopique électronique selon la revendication 4, dans lequel les pixels sont groupés en un premier groupe (52) et un deuxième groupe (53), et le premier groupe est utilisé pour capturer l'image de l'objet, et le deuxième groupe est utilisé pour obtenir la valeur de sortie d'obscurité, et le deuxième groupe est protégé par un film formant écran à la lumière.

9. Système endoscopique électronique selon la revendication 8, dans lequel la valeur de sortie d'obscurité est une moyenne de valeurs de pixel d'obscurité prises à partir des pixels respectifs dans le deuxième groupe.

10. Système endoscopique électronique selon la revendication 9, dans lequel la valeur de sortie d'obscurité est une moyenne des valeurs de pixel d'obscurité des N trames.

11. Système endoscopique électronique selon la revendication 4, dans lequel la mémoire est une mémoire de table (39) mémorisant les informations de conversion de température.

12. Système endoscopique électronique selon la revendication 11, dans lequel la température correspondant à une valeur de sortie d'obscurité qui n'est pas contenue dans la mémoire de table est calculée en utilisant une interpolation.

13. Système endoscopique électronique selon la revendication 4, dans lequel le contrôleur de quantité de lumière fixe une limite supérieure à la quantité de lumière de la lumière d'éclairage en fonction de la température, et le contrôleur de quantité de lumière commande la quantité de lumière de la lumière d'éclairage pour qu'elle ne dépasse pas la limite supérieure.

14. Système endoscopique électronique selon la revendication 13, dans lequel la limite supérieure comprend une première limite supérieure avec une quantité de lumière élevée et une deuxième limite supérieure avec une faible quantité de lumière, et le contrôleur de quantité de lumière fixe la deuxième limite supérieure en tant que limite supérieure lorsque la température dépasse une première température qui est une température élevée, et le contrôleur de température fixe la première limite supérieure en tant que limite supérieure lorsque la température est inférieure ou égale à une deuxième température qui est une température faible.
